# EUROPÄISCHE PATENTSCHRIFT

(11) **EP 1 067 385 B1**
(45) Veröffentlichungstag und Bekanntmachung des Hinweises auf die Patenterteilung: **12.02.2003**
(21) Anmeldenummer: 00112457.7
(22) Anmeldetag: 10.06.2000
(51) Int. Cl.: G01N 33/579, C12Q 1/04

(54) **Verfahren zur Quantifizierung der Beladung von Probenkörpern mit Biofilm**
Method for the quantification of the biofilm load on sample bodies
Méthode pour la quantification de la charge en biofilm sur les corps échantillons

(30) Priorität: 07.07.1999 DE 19931276
(43) Veröffentlichungstag der Anmeldung: 10.01.2001
(73) Patentinhaber: Seipp, Hans-Martin, Dr. med., 35039 Marburg (DE)
(72) Erfinder: Seipp, Hans-Martin, Dr. med., 35039 Marburg (DE)
(74) Vertreter: von Bülow, Tam, Dr.

(56) Entgegenhaltungen:
- EP-A- 0 291 856
- WO-A-97/33972
- US-A- 5 910 420
- US-A- 5 928 889
- RIOUFOL C ET AL: "Quantitative determination of endotoxins released by bacterial biofilms." JOURNAL OF HOSPITAL INFECTION, Bd. 43, Nr. 3, November 1999 (1999-11), Seiten 203-209, XP000996287 ISSN: 0195-6701

## Beschreibung

Die Erfindung betrifft ein Verfahren zur Quantifizierung der Beladung von Probenkörpern mit Biofilm gemäß dem Oberbegriff des Patentanspruches 1 bzw. 13.

Ein derartiges Verfahren ist aus der US 5,591,628 bekannt.

Ein zentrales Problem in der Medizin und Technik ist die Reinigung, Desinfizierung bzw. Sterilisierung von Geräten, insbesondere von hydraulischen Komponenten, wie z.B. Dialyseleitungen. Ein Vergleich unterschiedlicher Behandlungsverfahren bzw. Reinigungs- und Aufbereitungsprozesse hinsichtlich deren Antibiotika-, Desinfektions- bzw. Sterilisationswirkungen setzt eine Quantifizierbarkeit der Kontamination der zu behandelnden Objekte vor und nach der Behandlung voraus.

Auf Bauteiloberflächen hydraulischer Systeme entstehen häufig unerwünschte Anlagerungen von Mikroorganismen, die als "Biofilm" bezeichnet werden. Die Entstehung eines solchen Biofilms ist ein kontinuierlicher Vorgang, der als "Biofouling" bezeichnet wird [FLEMMING, HANS-CURT: Biofouling bei Membranprozessen, Willy-Hager-Stiftung Stuttgart, Springer Verlag, ISBN 3-540-58596-6] und der aus mehreren Einzelschritten besteht, nämlich
- Konditionierung der zu besiedelnden Oberfläche
- Adhäsion der Mikroorganismen
- Mikrokolonisation
- Weiterentwicklung des reifen Biofilms und
- Ablösung einzelner Bestandteile.

Biofilme werden beispielsweise durch Bakterien, insbesondere durch gram-negative aber auch durch gram-positive Bakterien, durch Fadenpilze und Schleimpilze gebildet. Bei hydraulischen Systemen, wie z.B. Dialyseleitungen, sowie in der Sterilisationstechnik stellen insbesondere die gram-negativen Bakterien ein hohes Gefahrenpotential dar, da in den Zellwänden solcher Bakterien Endotoxine enthalten sind, die beim Menschen neben Fieber zahlreiche weitere pathophysiologische Wirkungen hervorrufen können. Bei Trink- und Abwasseraufbereitungsanlagen, wie z.B. bei Umkehrosmose-Anlagen oder Ionentauscherharzen, können die Biofilme die Anlagenleistung massiv beeinträchtigen, was sich z.B. bei Umkehrosmose-Anlagen darin äußert, daß ca. 30 % der Betriebskosten für Gegenmaßnahmen zur Biofilmbildung aufgewendet werden. Auch in der Halbleiter- bzw. Reihenraumtechnik verursachen Biofilme in Wasserleitungssystemen erhebliche Kosten. Die Geräte bzw. Anlagen müssen daher regelmäßig und zuverlässig gereinigt werden, weshalb es wünschenswert ist, verschiedene Behandlungs- bzw. Aufbereitungsverfahren hinsichtlich ihrer Wirkungseffizienz miteinander zu vergleichen.

Zur Beseitigung von Biofilmen werden verschiedene Biozide verwendet, wie z.B. das Desinfektionsmittel Formaldehyd, Peroxide, Chlorverbindungen, Laugen, Säuren, insbesondere Citronensäure, Tenside, Enzyme, Natriumlaurylsulfat (SDS), Natriumbisulfit, etc., sowie physikalische Verfahren, wie z.B. thermische Desinfektion oder Ultraschall-Desinfektion, Bestrahlung mit ultraviolettem Licht etc., aber auch Antibiotika gegenüber Biofilmen auf transplantiertem oder implantierten Material sowie in Infektionsherden. Derzeit wird ferner erforscht, inwiefern Biofouling durch vorgeschaltete Biofilter minimiert werden kann, welche die Nährstoffzufuhr zu Biofilm-gefährdeten Komponenten reduzieren.

Zum Nachweis von Biofilmen gibt es verschiedene Verfahren, die sich in quantitative, semi-quantitative und qualitative Nachweisverfahren unterteilen lassen.

Die eingangs erwähnte US 5,591,628 beschreibt ein Verfahren, bei dem mittels des LAL-Tests gram-negative Bakterien, Endotoxine und Lipopolysaccharide nachgewiesen werden. Der LAL-Test basiert auf einem Limulus-Amöbozyt-Lysat, das aus der Hämolymphe der Hufeisenkrappe gewonnen wird. Eine Lyse der Amöbozyten setzt eine Substanz frei, die mit Endotoxin unter der Bildung eines Gels reagiert. Diese Reaktion zwischen Limulus-Amöbozyten-Lysat und Endotoxin wird als LAL-Test bezeichnet. Es sind mehrere Ausführungsbeispiele erläutert, die einen qualitativen und quantitativen Endotoxin-Nachweis ermöglichen.

Die US 5,605,806 beschreibt ein Reagenz zum Nachweis von Endotoxinen gram-negativer Bakterien, die in einer biologischen Probe, wie Blut, Urin oder Gehirnflüssigkeit, enthalten sind.

Die DE 31 12 441 C2 beschreibt eine Vorrichtung zur Vorbereitung der qualitativen und quantitativen Bestimmung von bakteriellen Endotoxinen in wäßrigen Lösungen für einen Nachweis von Pyrogenen bei der Qualitätskontrolle von Ausgangsmaterialien, Vor- und Zwischenprodukten bei der Herstellung von Injektions- und Infusionslösungen, wobei der LAL-Test angewandt wird.

Die US 5,316,911 beschreibt ebenfalls ein Verfahren zum Nachweis von Endotoxinen mittels des LAL-Tests.

Die DE 195 48 300 A1 beschreibt ein elektro-chemisches Verfahren zur Bestimmung der Aktivität von Biofilmen auf Festkörperoberflächen, wobei das Redoxpotential zwischen der Festkörperoberfläche und dem Biofilm gemessen wird.

Ferner sind Verfahren zum Anzüchten von Biofilmen bekannt, die aus gram-negativen Bakterien bzw. Pilzen bestehen, und zwar aus:
- Datenbank MEDLINE bei STN; AN 96044935 MEDLINE zu: Biofilms produced by Pseudomonas aeruginosa and by Staphylococcus aureus on model medical devices [comment]. Comment on: Acta Microbiol. Immunol. Hung. 1995;42(2):215-9 Ketyi, I. Acta Microbiologica et Immunologica Hungarica, (1995) 42 (2) 221-227;
- Datenbank BIOSIS bei STN; AN 1992:53358 BIOSIS zu: Growth characteristics and expression of iron-regulated outer-membrane proteins of chemostat-grown biofilm cells of Pseudmonas aeruginosa. Anwar, H. et al., Can. J. Microbiol., (1991) 37 (10), 737-743;
- Datenbank BIOSIS bei STN; AN 1998:453587 BIOSIS zu: Biofilms on indwelling urethral catheters produce quorum-sensing signal molecules in situ and in vitro. Stickler, D.J. et al., Applied and Environmental Microbiology, (Sept., 1998) Vol. 64, No. 9, pp. 3486-3490,
- Datenbank BIOSIS bei STN; AN 1995:217272 BIOSIS zu: Bacterial biofilm growth on ciprofloxacin treated urethral catheters. Stickler, D.J. et al., Cells and Materials, (1994) Vol. 4, No. 4, pp. 387-396,
- Datenbank BIOSIS bei STN; AN 1985:340646 BIOSIS zu: Tobramycin resistance of Pseudmonas aeruginosa cells growing as a biofilm on urinary catheter material. Nickel, J.C. et al., Antimicrob. Agents Chemother., (1985) 27 (4), 619-624,
- Datenbank BIOSIS bei STN; AN 1997:202828 BIOSIS zu: Activity of toluene-degrading Pseudomonas putida in the early growth phase of a biofilm for waste gas treatment. Pedersen, A.R. et al., Biotechnology and Bioengineering, (1997) Vol. 54, No. 2, pp. 131-141,
- Datenbank BIOSIS bei STN; AN 1994:167287 BIOSIS zu: Growth and in situ detection of a pathogenic Escherichia coli in biofilms of a heterotrophic waterbacterium by use of 16S- and 23S-rRNA-directed fluorescent oligonucleotide probes. Szewzyk, U. et al., FEMS (Federation of European Microbiological .Societies) Microbiology Ecology, (1994) Vol. 13, No. 3, pp. 169-175,
- Datenbank BIOSIS bei STN; AN 1996:331637 BIOSIS zu: Spatial variations in growth rate within Klebsiella pneumoniae colonies and biofilm. Wentland, E.J. et al., Biotechnology Progress, (1996) Vol. 12, No. 3, pp. 316-321,
- Datenbank BIOSIS bei STN; AN 1997:436004 BIOSIS zu: Effects of salivary or serum pellicles on the Candida albicans growth and biofilm formation on soft lining materials in vitro. Nikawa, H. et al., Journal of Oral Rehabilitation, (1997) Vol. 24, No. 8, pp. 594-604.

Bei einem quantitativen Verfahren wird die Konzentration von Mikroorganismen in einem strömenden Medium untersucht, z.B. von Wasser, das in einer Leitung strömt. Mit diesem Verfahren ist jedoch keine quantifizierbare Korrelation zwischen der im strömenden Medium ermittelten Konzentration der Mikroorganismen und der tatsächlich vorhandenen Biofilmbelastung an der Bauteiloberfläche herstellbar. Bei dieser mikrobiologischen Keimzahlbestimmung werden nämlich lediglich die vermehrungsfähigen Mikroorganismen auf dem vorhandenen Nährboden erfaßt, welche zufällig, jedoch nicht repräsentativ, aus dem Biofilm herausgelöst werden, wohingegen Spezies mit anderen Nährstoffansprüchen sowie die im Biofilm verbleibenden, lebenden oder abgestorbenen Mikroorganismen über Probenahmen des durchströmenden Wassers nicht nachweisbar sind.

Ferner gibt es Forschungsvorhaben zum quantitativen Nachweis von Elektronentransportvorgängen, die jedoch ebenfalls ausschließlich die Zellen vermehrungsfähiger, d.h. lebender Mikroorganismen erfassen.

Weiter ist es bekannt, bei medizinischen hydraulischen Systemen die Endotoxinaktivität zu ermitteln, um daraus die Menge der im untersuchten Medium vorhandenen Fiebererreger zu bestimmen. Ein quantitativer Zusammenhang zwischen der im strömenden Medium herrschenden Endotoxinaktivität und der tatsächlichen Belastung des hydraulischen Systems mit Biofilm ist auch hiermit nicht möglich.

Ferner wurden die Audioradiographie sowie die Epifluoreszenzmikroskopie sowie Kombinationen beider Verfahren als Nachweisverfahren eingesetzt, die jedoch ebenfalls nur einen Nachweis vermehrungsfähiger, nicht jedoch abgestorbener Mikroorganismen des Biofilms ermöglichen.

Ein bekanntes semiquantitatives Nachweisverfahren verwendet zur Bestimmung der Gesamtzahl im Biofilm vorhandener Zellen Färbemittel, wie z.B. Acridinorange, wobei der Verfärbungsgrad Aufschluß über die Zellanzahl geben soll.

Ein qualitativer, makroskopischer Nachweis von Biofilm ist mit einer "Brennprobe" möglich, bei der kleine Mengen Biofilm über einer offenen Flamme erhitzt werden und aus dem Geruch verbrannten Proteine auf den Biofilm rückgeschlossen wird.

Für die Bestimmung der Wirkung von Desinfektionsmitteln wurden von Kinniment und Wimpenny in einem speziellen Fermenter Biofilme hergestellt, die mittels scharfer Klingensysteme mechanisch entnommen wurden, wobei auch hier ausschließlich die Anzahl lebender Mikroorganismen bestimmt wurde.

Die oben erwähnten für den Menschen gefährlichen Endotoxine werden jedoch nicht von lebenden Bakterien ausgeschieden, sondern erst durch Lyse aus der Zellwand abgestorbener, gram-negativer Bakterien freigesetzt.

Derzeit gibt es kein exaktes Nachweisverfahren zur Quantifizierung der Beladung von Oberflächen mit Biofilm. Folglich ist eine Beurteilung von Biofilm-Flächenbelastungen nicht möglich. Eine quantitativ vergleichende Bewertung verschiedener chemischer, physikalischer und antibiotischer Verfahren zur Beseitigung von Biofilm, d.h. von Behandlungsverfahren, wie Reinigungs-, Desinfektions-, Antibiotika- und Chemotherapeutika-Verfahren und eine Untersuchung des Einflusses verschiedener Materialien, wie z.B. Kunststoff, Glas, Metalle und deren Legierungen, auf die Vermeidung bzw. die Entstehung von Biofilm ist daher ebenfalls nicht möglich.

Aufgabe der Erfindung ist es, ein Verfahren zur Quantifizierung der Beladung von Probenkörpern und von technischen Systemen mit Biofilm anzugeben.

Diese Aufgabe wird durch die Merkmale der Patentansprüche 1 und 13 gelöst. Vorteilhafte Ausgestaltungen und Weiterbildungen der Erfindung sind den Unteransprüchen zu entnehmen.

Der Erfindung liegt die Idee zugrunde, zunächst einen Probenkörper mit einer vorgegebenen homogenen Biofilmbeladung herzustellen. Anschließend wird der vorgegebenen Beladung des Probenkörpers mit Biofilm durch Anwendung eines chemischen Nachweisverfahrens ein Beladungsreferenzwert zugeordnet. Mit demselben Nachweisverfahren wird für einen zweiten Probenkörper mit unbekannter Biofilmbeladung ein Beladungswert ermittelt. Ein Vergleich des Beladungswerts des zweiten Probenkörpers mit dem Referenzwert des ersten Probenkörpers ermöglicht eine Quantifizierung der Biofilmbeladung des zweiten Probenkörpers.

Zunächst wird die Herstellung von Probenkörper mit vorgegebener homogener Biofilmbeladung und anschließend das chemische Nachweisverfahren näher erläutert.

Der Herstellvorgang kann in mehrere Phasen unterteilt werden, nämlich eine fakultative Adaptationsphase, eine fakultative Reisolierungsphase sowie eine notwendige Inkubationsphase und eine notwendige Erntephase.

In der Adaptationsphase werden Mikroorganismen, wie gram-negative Bakterien (Pseudomonas aeruginosa) oder Pilze, in ein Nährmedium, z.B. eine zell-physiologische Mineralsalzlösung, in einer vorgegebenen Koloniezahl eingebracht. Diese Koloniezahl wird in der Einheit "Kolonie-bildende-Einheit pro Milliliter [KBE/ml]" angegeben und ist vorzugsweise größer als 10⁶. Das Nährmedium wird dabei auf einen Probenkörper aufgebracht, der aus einem vorgegebenen Material hergestellt ist. Die Mikroorganismen, wie z.B. gram-negative Bakterien und Pilze (z.B. Aspergillus niger) werden dann über mehrere Stunden bis Wochen in dem Nährmedium an das Material des Probenkörpers adaptiert. In manchen Fällen kann die Adaptationsphase fortgelassen werden, wenn die Mikroorganismen schon an das Material adaptiert sind.

Nach Abschluß der Adaptationsphase werden die inkubierten Mikroorganismen als Einzelkolonien isoliert und das in der Adaptationsphase verwendete Probenkörpermaterial wird als Abfall entsorgt.

Vor Beginn der Inkubationsphase werden die isolierten Einzelkolonien der Mikroorganismen zunächst im Rahmen einer laborüblichen Kultur auf eine Koloniezahl von > 10⁸ KBE/ml angezüchtet. Anschließend werden die angezüchteten Mikroorganismuskolonien zentrifugiert, z. B. mit einer Drehzahl von 3000 s⁻¹ und gewaschen, wobei diese beiden Vorgänge beispielsweise zweimal wiederholt werden können. Nach der Zentrifugation und dem Waschen werden die Mikroorganismen in Nährlösung suspendiert. Die Nährlösung bringt man dann auf die Oberfläche eines gereinigten Probenkörpers auf, der aus dem gleichen Material hergestellt ist, wie der in der Adaptationsphase verwendete Probenkörper. Vorzugsweise erfolgt das Aufbringen der Nährlösung durch ein Überströmen, wobei die Überströmgeschwindigkeit der Nährlösung vorzugsweise gering ist und z.B. kleiner als 0,05 m/sec. Während der nun folgenden Inkubationsphase können zu verschiedenen Zeitpunkten Teilproben aus der Oberfläche und des inkubierten Probenkörpers entnommen werden, um das Entwicklungsstadium der Mikroorganismen, d.h. die aktuell vorhandene Flächenbeladung mit Biofilm, mit einem Nachweisverfahren zu untersuchen.

Nach Erreichen einer gewünschten homogenen Biofilmbeladung wird die Inkubationsphase beendet, indem die Oberfläche des Probenkörpers mit steriler Lösung gespült wird. In dieser "Erntephase" kann der Probenkörper mit bekannter vorgegebener Biofilmbeladung bzw. einer entsprechenden Endotoxin- (gram-negative Bakterien) bzw. 1,3-β-D-Glucan (Pilze) -aktivität in mehrere gleichgroße Probenkörper zerschnitten werden.

Die erhaltenen Probenkörper können dann über mehrere Monate gelagert werden. Die Probenkörper können hierfür entweder getrocknet oder bei z.B. -20°C tiefgekühlt gelagert werden.

Im folgenden wird das Nachweisverfahren zur Quantifizierung der Biofilmbeladung von Probenkörpern erläutert.

In einem ersten Schritt wird der durch Mikroorganismen gebildete Biofilm durch ein Lösungsmittel aufgelöst. Als Lösungsmittel können Substanzen verwendet werden, die geeignet sind, native und/oder nach dem oben beschriebenen Verfahren auf einem Probenkörper gezüchtete Biofilme vollständig aufzulösen und zwar so, daß das in den gram-negativen Bakterien enthaltene Endotoxin oder Zellwandbestandteile von Pilzen weitestgehend oder vollständig freigesetzt wird, ohne daß deren chemische Aktivität hierdurch wesentlich reduziert wird. Hierfür geeignet ist beispielsweise eine Natriumhypochlorit-Lösung. Die Aktivchlorkonzentration im alkalischen Milieu (pH > 11) ist beispielsweise kleiner als 5 g Cl₂/l, vorzugsweise 1,2 g bis 1,8 g Cl₂/l. Solche Lösungen können ferner unter Verwendung von Kalilauge mit einer Konzentration von 108 g/l, Kaliumtriphosphat mit einer Konzentration von 300 g/l und zur Stabilisierung mit Kaliwasserglas mit einer Konzentration von 210 g/l - 35 Bé hergestellt werden.

Die Auflösung des Biofilms bzw. deren Mikroorganismen (Lyse) erfolgt mit einem Lösungsmittelüberschuß, der so groß ist, daß der Auflösungsprozeß bei Raumtemperatur innerhalb eines Zeitraumes von weniger als 60 min beendet ist.

Nach Beendigung der Biofilmauflösung bzw. Zell-Lyse kann eine stöchiometrische Neutralisation der Natriumhypochlorit-Lösung mit einem Neutralisierungsmittel erfolgen, wie z.B. einer Natriumthiosulfat-Lösung.

Aus der neutralisierten, die Endotoxine bzw. Zellwandbestandteile von Pilzen enthaltenden Lösung werden dann mehrere - z.B. dekadische - Verdünnungsstufen hergestellt. Die Verdünnungsstufen werden dann in einem - z.B. kinetisch-turbidimetrischen - Messverfahren untersucht, wodurch die enthaltenen Endotoxinmengen und Zellwandbestandteile ermittelt werden können. Anhand der ermittelten Mengen kann die ursprünglich auf dem verwendeten Probenkörper vorhandene Biofilmbeladung quantifiziert werden. Als Nachweisverfahren zur Bestimmung der Endotoxin- und Zellwandmenge von Pilzen eignet sich der bekannte Limulus-Amöbozyten-Lysat-Test (vgl. Römpp, Chemielexikon, Georg Thieme Verlag, 1995).

Die Erfindung kann beispielsweise zur qualitativen Bewertung von Behandlungsverfahren, wie z.B. der Reinigungs-, Desinfektions-, Antibiotika- bzw. Sterilisationsgüte einer Reinigungsmaschine bzw. zum qualitativen Vergleich mehrerer Behandlungsverfahren verwendet werden, wobei folgendermaßen vorgegangen werden kann:

Zunächst wird für eine statistisch signifikante Bewertung ausreichende Anzahl von mit Biofilm beladenen Probenkörpern ein Referenzwert für die Biofilmbeladung ermittelt. Hierzu werden die Probenkörper nach dem oben beschriebenen Verfahren hergestellt und gemäß dem erläuterten Nachweisverfahren zur Bestimmung der Endotoxin- bzw. 1,3-β-D-Glucan-Aktivität untersucht.

Anschließend werden andere Probenkörper mit der gleichen Biofilmbeladung einem zu beurteilenden Behandlungsverfahren unterzogen und während oder danach mittels dem Nachweisverfahren auf die verbliebene Biofilmrestbeladung untersucht. Die Effizienz bzw. Güte des zu beurteilenden Behandlungsverfahrens (Reinigung, Desinfektion, Antibiotika, Sterilisation) ergibt sich dann durch Vergleich des erhaltenen Beladungswerts mit dem Referenzwert für die Endotoxinaktivität der in vorgegebener Weise hergestellten Probenkörper.

Die Erfindung ermöglicht somit einerseits eine standardisierte Herstellung und langfristige Lagerung einer beliebigen Anzahl von mit Biofilm beladenen Probenkörpern (Charge), die homogen mit Mikroorganismen einer oder mehrerer gram-negativer Bakterienspezies oder Pilzen beschichtet sind. Die Schichtdicke des Biofilms kann dabei durch die prozeßtechnisch steuerbare Inkubationsdauer variiert werden und kann während der laufenden Inkubationszeit meßtechnisch durch Messung der bereits gebildeten Endotoxinmenge nachgewiesen werden. Somit können Materialoberflächen homogen mit beliebigen Biofilm-Schichtdicken beladen werden.

Die Endotoxinaktivität der nach abgeschlossener Herstellung einer Charge homogen mit Biofilm beladenen Materialoberflächen stellt eine Referenz- bzw. Bezugsgröße für die nach Einwirkung eines zu bewertenden Behandlungsverfahrens verbliebene Endotoxinaktivität dar.

Im Gegensatz zum Stand der Technik basiert das den Herstellungsprozeß kontrollierend begleitende Nachweisverfahren auf einer vollständigen Entfernung des Biofilms vom Probenkörper und einer vollständigen Zersetzung des Biofilms sowie auf einer chemischen Lyse der einzelnen Mikroorganismen von den Oberflächen der Probekörper, beispielsweise durch Natriumhypochlorit.

Das Verfahren kann auch ohne die speziellen Prüfkörper verwendet werden, nämlich dort, wo sich Biofilme natürlich ausbilden, beispielsweise in Dialysegeräten, Geräten zur Umkehrosmose, Wasseraufbereitungsanlagen oder Rohrleitungen bei der Chip-Herstellung. Es wird dann parallel bzw. begleitend zu einem Reinigungsprozeß ausgeführt. Beim Reinigungsprozeß wird Hypochlorit zugegeben und untersucht, ob mit dem Nachweisverfahren Biofilmreste nachzuweisen sind. Ist dies der Fall, so wird nach einer weiteren Reinigungsdauer erneut Hypochlorit zugegeben und dieser Schritt gegebenenfalls mehrfach solange wiederholt, bis keine Biofilmreste mehr nachweisbar sind. Dieser Reinigungsprozeß mit Nachweisverfahren wird in vorgegebenen Zeiträumen, beispielsweise drei oder sechs Monaten, wiederholt. Hieraus läßt sich dann auch eine Zeitspanne bestimmen, die für Reinigungsintervalle zweckmäßig ist. Bisher werden nämlich manche Anlagen unabhängig von ihrer Biofilmbeladung in regelmäßigen Abständen behandelt bzw. gereinigt, was, da es mehr oder weniger willkürlich erfolgt, zu häufig oder auch zu selten sein kann. Mit dem jetzigen Verfahren können dagegen die Intervalle präziser der tatsächlichen Biofilmbeladung angepaßt werden.

Ein wesentlicher Vorteil der Erfindung ist, daß die Endotoxinaktivität des aufzulösenden Biofilms durch die chemische Lyse und die anschließende Neutralisation nicht relevant beeinflußt wird und deshalb mit Standardverfahren, wie z.B. dem Limulus-Amöbozyten-Lysat-Test (LAL-Test), untersucht werden kann.

Im Gegensatz zum Stand der Technik können somit nicht nur die vermehrungsfähigen, sondern auch die bereits abgestorbenen Mikroorganismen mittelbar über die aus deren Zellwänden freigesetzte Endotoxin- bzw. 1,3-β-D-Glucan-Aktivität meßtechnisch quantifiziert werden.

Die erläuterte Kombination der Biofilm-Herstellung und des Biofilm-Nachweisverfahrens erlaubt es, alle im Zusammenhang mit dem Auftreten von Biofilmen oder Biofouling relevanten Behandlungsverfahren, d.h. Reinigungs-, Desinfektions- bzw. Sterilisationsverfahren, hinsichtlich ihrer Wirksamkeit zu vergleichen und zu bewerten. Weiter können auch alle in hydraulischen Systemen verwendeten Materialien sowie Implantat- und Transplantatoberflächen hinsichtlich ihrer Biofilm begünstigenden oder Biofilm inhibierenden Eigenschaften beurteilt werden.

## Patentansprüche

1. Verfahren zur Quantifizierung der Beladung von Probenkörperoberflächen mit durch gram-negative Bakterien oder Pilze gebildeten Biofilm, wobei die auf dem Probenkörper vorhandene Biofilmbeladung durch Bestimmung der im Biofilm enthaltenen Endotoxin- oder 1,3-β-D-Glucan-Menge durch Anwendung des Limulus-Amöbozyten-Lysat-Testverfahrens ermittelt wird, **dadurch gekennzeichnet,**
**daß** zuvor folgende Schritte durchgeführt werden:
a) Vollständiges Auflösen des dem Probenkörper anhaftenden Biofilms mit einem Lösungsmittel, das in den Mikroorganismen des Biofilms enthaltene Endotoxine und/oder 1,3-β-D-Glucan vollständig freisetzt und dabei die chemische Aktivität dieser Stoffe im wesentlichen unbeeinflußt läßt;
b) Entnahme von Flüssigkeitsproben aus dem Lösungsmittel, in dem der Biofilm gelöst ist;
c) Herstellen unterschiedlicher Verdünnungsstufen aus den entnommenen Flüssigkeitsproben, wobei zur Quantifizierung der Biofilmbeladung mit den Verdünnungsstufen der Limulus-Amöbozyten-Lysat-Test durchgeführt wird.

2. Verfahren nach Anspruch 1, **dadurch gekennzeichnet, daß** die zur Auflösung verwendete Menge Lösungsmittel mit einem Neutralisierungsmittel stöchiometrisch neutralisiert wird.

3. Verfahren nach Anspruch 1 oder 2, bei dem
a) zwei Probenkörper mit gleicher Biofilmbeladung verwendet werden;
b) die Beladung eines der beiden Probenkörper quantifiziert und dieser Beladung ein Referenzwert zugeordnet wird;
c) der andere Probenkörper einem zu beurteilenden Behandlungsverfahren unterzogen wird;
d) nach der Behandlung die Beladung des anderen Probenkörpers quantifiziert wird; und
e) ein dieser Beladung entsprechender Wert mit dem Referenzwert verglichen wird.

4. Verfahren nach einem der Ansprüche 1 bis 3, wobei der bzw. die mit Biofilm beladenen Probenkörper mit folgenden Schritten hergestellt werden:
a) Adaptieren gram-negativer Bakterien bzw. von Pilzen an ein Probenkörpermaterial über einen Zeitraum von mehreren Stunden bis Wochen durch Inkubation in einem Nährmedium;
b) Trennen der inkubierten Bakterien bzw. Pilze von dem zum Adaptieren verwendeten Probenkörpermaterial und Isolieren einzelner Bakterienkolonien aus dem Biofilm;
c) Anzüchten der einzelnen Bakterien- bzw. Pilzkolonien auf eine Konzentration Kolonie-bildender-Einheiten, die größer als 10⁸/ml ist;
d) Aufbringen der angezüchteten Bakterien bzw. Pilze auf einen sterilisierten Probenkörper;
e) Inkubation der Bakterien bzw. Pilze auf dem Probenkörper über mehrere Stunden bis Wochen auf eine vorgegebene Biofilm-Beladung mit einer homogenen physikalischen Verteilung von Zellen im Biofilm; und
f) Spülen des Probenkörpers mit Endotoxin- und β-Dglucanfreier, frischer, steriler Lösung.

5. Verfahren nach Anspruch 4, **dadurch gekennzeichnet,**
**daß** die Konzentration Kolonie-bildender-Einheiten größer als 10⁶/ml ist.

6. Verfahren nach Anspruch 4 oder 5, **dadurch gekennzeichnet,**
**daß** die Mikroorganismen vor dem Aufbringen in einem Nährmedium suspendiert werden.

7. Verfahren nach einem der Ansprüch 4 bis 6, **dadurch gekennzeichnet,**
**daß** während der Dauer des Spülens zu verschiedenen Zeitpunkten Teilproben aus der Oberfläche des inkubierten Probenkörpers herausgeschnitten werden und daß diese Teilproben durch Anwendung eines Endotoxin- bzw. 1,3-β-D-Glucan-Nachweisverfahrens auf die aktuell vorhandene Flächenbeladung mit Biofilm untersucht werden.

8. Verfahren nach einem der Ansprüche 4 bis 7, **dadurch gekennzeichnet,**
**daß** das in Anspruch 4, Schritt e) genannte Aufbringen durch ein Überströmen des Probenkörpers mit einer Geschwindigkeit von weniger als 0,5 m/sec und vorzugsweise weniger als 0,05 m/sec erfolgt.

9. Verfahren nach einem der Ansprüche 4 bis 8, **dadurch gekennzeichnet,**
**daß** im Anschluß an Schritt f) des Anspruches 4 der Probenkörper in mehrere Probenkörper zerteilt wird.

10. Verfahren nach einem der Ansprüche 4 bis 9, **dadurch gekennzeichnet,**
**daß** fertig hergestellte Probenkörper getrocknet und in trockener Form gelagert werden.

11. Verfahren nach einem der Ansprüche 4 bis 10, **dadurch gekennzeichnet,**
**daß** die fertig hergestellten Probenkörper tiefgekühlt gelagert werden.

12. Verfahren nach Anspruch 11, **dadurch gekennzeichnet,**
**daß** die Probenkörper auf -20°C tiefgekühlt werden.

13. Verfahren zur Quantifizierung der Beladung von natürlich mit Biofilmen belegten Oberflächen, wobei die auf den Oberflächen vorhandene Biofilmbeladung durch Bestimmung der im Biofilm enthaltenen Endotoxin- oder 1,3-β-D-Glucan-Menge durch Anwendung des Limulus-Amöbozyten-Lysat-Testverfahrens ermittelt wird, wobei zuvor folgende Schritte durchgeführt werden:
a) Vollständiges Auflösen des an der Oberfläche des Systems anhaftenden Biofilms mit einem Lösungsmittel, das in den Mikroorganismen enthaltene Endotoxine oder 1,3-β-D-Glucane vollständig freisetzt und dabei die chemische Aktivität dieser Stoffe im wesentlichen unbeeinflußt läßt; und
b) Entnahme von Flüssigkeitsproben aus der Menge der zugesetzten Lösungsmittel, wobei mit den Flüssigkeitsproben zur Quantifizierung der Biofilmbeiadung der Limulus-Amöbozyten-Lysat-Test durchgeführt wird.

14. Verfahren nach Anspruch 13, **dadurch gekennzeichnet,**
**daß** das Verfahren solange wiederholt wird, bis die Biofilmbeladung vollständig aufgelöst ist, d.h. bis keine Endotoxin- und/oder 1,3-β-D-Glucan-Mengen mehr nachweisbar sind.

15. Verfahren nach einem der Ansprüche 1 bis 14, **dadurch gekennzeichnet,**
**daß** als Lösungsmittel Natriumhypochlorit verwendet wird.

16. Verfahren nach einem der Ansprüche 1 bis 15, **dadurch gekennzeichnet,**
**daß** bei der Auflösung des Biofilms mit einem Lösungsmittelüberschuß gearbeitet wird, so daß der Auflösungsprozeß bei Raumtemperatur innerhalb einer Zeitdauer von weniger als 60 min beendet ist.

17. Verfahren nach einem der Ansprüche 1 bis 16, **dadurch gekennzeichnet,**
**daß** als Neutralisierungsmittel Natriumthiosulfat verwendet wird.

## Claims

1. A method of quantifying the loading of sample surfaces with biofilm formed by gram-negative bacteria or fungi, wherein the biofilm load on the sample is established by determining the amount of endotoxins or 1,3-β-D-glucan contained in the biofilm by means of limulus-amoebocyte-lysate testing, **characterised in that** the following steps are carried out beforehand:
a) complete dissolution of the biofilm adhering to the sample using a solvent which completely releases endotoxins and/or 1,3-β-D-glucan contained in the micro-organisms of the biofilm and leaves the chemical activity of these substances substantially unaffected;
b) withdrawal of liquid samples from the solvent in which the biofilm has been dissolved;
c) preparation of different dilution phases from the withdrawn liquid samples, the limulus-amoebocyte-lysate test being carried out with the dilution phases for quantification of the biofilm load.

2. A method according to claim 1, **characterised in that** the amount of solvent used for dissolution is neutralised stoichiometrically with a neutralising agent.

3. A method according to claim 1 or 2, in which
a) two samples with the same biofilm loading are used;
b) the loading of one of the two samples is quantified and a reference value is assigned to this load;
c) the other sample undergoes a treatment process to be evaluated;
d) after treatment, the loading of the other sample is quantified; and
e) a value corresponding to this load is compared with the reference value.

4. A method according to any one of claims 1 to 3, wherein the sample or samples loaded with biofilm are prepared by the following steps:
a) adapting gram-negative bacteria or fungi to a sample material over a period of several hours to weeks by incubation in a nutrient medium;
b) separating the incubated bacteria or fungi from the sample material used for adaptation and isolating individual bacterial colonies from the biofilm;
c) cultivating the individual bacterial or fungal colonies until reaching a concentration of colonising units greater than 10⁸/ml;
d) applying the cultivated bacteria or fungi to a sterilised sample;
e) incubating the bacteria or fungi on the sample over several hours to weeks until reaching a predetermined biofilm loading with a homogeneous physical distribution of cells in the biofilm; and
f) rinsing the sample with a fresh sterile solution free of endotoxins and β-D-glucan.

5. A method according to claim 4, **characterised in that** the concentration of colonising units is greater than 10⁶/ml.

6. A method according to claim 4 or 5, **characterised in that** the micro-organisms are suspended in a nutrient medium before application.

7. A method according to any one of claims 4 to 6, **characterised in that**, at different times during the rinsing period, sub-samples are cut out of the surface of the incubated sample and **in that** the current biofilm load on the surface of these sub-samples is tested by means of an endotoxin or 1,3-β-D-glucan detection process.

8. A method according to any one of claims 4 to 7, **characterised in that** the application process referred to in claim 4, step d) is carried out by a stream being passed over the sample at a rate of less than 0.5 m/sec and preferably less than 0.05 m/sec.

9. A method according to any one of claims 4 to 8, **characterised in that**, after step f) of claim 4, the sample is divided into a plurality of samples.

10. A method according to any one of claims 4 to 9, **characterised in that** ready prepared samples are dried and stored in dry form.

11. A method according to any one of claims 4 to 10, **characterised in that** ready prepared samples are stored in frozen form.

12. A method according to claim 11, **characterised in that** the samples are frozen to -20°C.

13. A method of quantifying the loading of surfaces naturally covered with biofilms, wherein the biofilm load on the surfaces is established by determining the amount of endotoxins or 1,3-β-D-glucan contained in the biofilm by means of limulus-amoebocyte-lysate testing, **characterised in that** the following steps are carried out beforehand:
a) complete dissolution of the biofilm adhering to the surface of the system using a solvent which completely releases endotoxins and/or 1,3-β-D-glucans contained in the micro-organisms of the biofilm and leaves the chemical activity of these substances substantially unaffected;
b) withdrawal of liquid samples from the quantity of added solvents, the limulus-amoebocyte-lysate test being carried out with the liquid samples for quantification of the biofilm load.

14. A method according to claim 13, **characterised in that** the method is repeated until the biofilm load has been dissolved completely, i.e. until no more endotoxins or 1,3-β-D-glucan are detectable.

15. A method according to any one of claims 1 to 14, **characterised in that** sodium hypochlorite is used as solvent.

16. A method according to any one of claims 1 to 15, **characterised in that** dissolution of the biofilm is carried out with excess solvent so that the dissolution process at room temperature is complete within a period of less than 60 mins.

17. A method according to any one of claims 1 to 16, **characterised in that** sodium thiosulphate is used as neutralising agent.

## Revendications

1. Méthode de quantification de la charge en biofilm formé par des bactéries Gram-négatives ou des champignons sur les surfaces d'un corps échantillon, la charge en biofilm présente sur le corps échantillon étant calculée en utilisant le test au lysat d'amoebocytes de limule (LAL) afin de déterminer la quantité d'endotoxine ou de 1,3-β-D-glucane contenue dans le biofilm, **caractérisée en ce qu'**on exécute auparavant les étapes suivantes :
a) dissolution complète du biofilm adhérant au corps échantillon, au moyen d'un solvant qui libère intégralement l'endotoxine et/ou le 1,3-β-D-glucane contenus dans les micro-organismes et qui préserve pour l'essentiel l'activité chimique de ces substances ;
b) prélèvement d'échantillons de liquide à partir du solvant dans lequel le biofilm est dissous ;
c) établissement de différents stades de dilution à partir des échantillons de liquide prélevés, le test au lysat d'amoebocytes de limule étant réalisé, selon les stades de dilution, pour quantifier la charge en biofilm.

2. Méthode selon la revendication 1, **caractérisée en ce que** la quantité de solvant utilisée pour la dissolution est neutralisée par un agent neutralisant selon les règles stoechiométriques.

3. Méthode selon la revendication 1 ou 2, dans laquelle
a) on utilise deux corps échantillons présentant la même charge en biofilm ;
b) on quantifie la charge de l'un des deux corps échantillons et l'on attribue à cette charge une valeur de référence ;
c) on soumet l'autre corps échantillon aux méthodes de traitement que l'on veut évaluer ;
d) après le traitement, on quantifie la charge de l'autre corps échantillon ; et
e) on compare à la valeur de référence la valeur correspondant à cette charge.

4. Méthode selon l'une des revendications 1 à 3, dans laquelle le ou les corps échantillons chargés en biofilm sont produits avec les étapes suivantes :
a) adaptation de bactéries Gram-négatives ou de champignons à un matériau de corps échantillon, pendant une période pouvant s'étendre de plusieurs heures à plusieurs semaines, par incubation dans un milieu de culture ;
b) séparation des bactéries ou des champignons incubés et du matériau de corps échantillon utilisé pour l'étape d'adaptation, et isolation de colonies individuelles de bactéries issues du biofilm ;
c) mise en culture des colonies individuelles de bactéries ou de champignons jusqu'à une concentration d'unités formant colonie supérieure à 10⁸/ml ;
d) application sur un corps échantillon stérilisé des bactéries ou des champignons mis en culture ;
e) incubation des bactéries ou des champignons sur le corps échantillon, pendant une période pouvant s'étendre de plusieurs heures à plusieurs semaines, jusqu'à atteindre une charge en biofilm prédéterminée, présentant une répartition physique homogène de cellules dans le biofilm ; et
f) rinçage du corps échantillon avec une solution stérile fraîche exempte d'endotoxine et de β-D-glucane.

5. Méthode selon la revendication 4, **caractérisée en ce que** la concentration d'unités formant colonie est supérieure à 10⁶/ml.

6. Méthode selon la revendication 4 ou 5, **caractérisée en ce que** avant l'étape d'application, on met en suspension les micro-organismes dans un milieu de culture.

7. Méthode selon l'une des revendications 4 à 6, **caractérisée en ce que** pendant le rinçage on découpe, à différents moments, des morceaux d'échantillons sur la surface du corps échantillon incubé, et l'on étudie la charge en biofilm présente à ces moments-là sur la surface de ces morceaux d'échantillons, en utilisant une méthode de détection de l'endotoxine ou du 1,3-β-D-glucane.

8. Méthode selon l'une des revendications 4 à 7, **caractérisée en ce que** dans la revendication 4, l'étape d) d'application s'effectue par un noyage du corps échantillon selon une vitesse inférieure à 0,5 m/sec et de préférence inférieure à 0,05 m/sec.

9. Méthode selon l'une des revendications 4 à 8, **caractérisée en ce que** à la suite de l'étape f) de la revendication 4, on divise le corps échantillon en plusieurs corps échantillons.

10. Méthode selon l'une des revendications 4 à 9, **caractérisée en ce que** les corps échantillons finis sont séchés et stockés sous forme sèche.

11. Méthode selon l'une des revendications 4 à 10, **caractérisée en ce que** les corps échantillons finis sont stockés à basse température.

12. Méthode selon la revendication 11, **caractérisée en ce que** les corps échantillons sont réfrigérés à - 20°C.

13. Méthode de quantification de la charge des surfaces recouvertes naturellement de biofilms, la charge en biofilm présente sur les surfaces étant calculée en utilisant le test au lysat d'amoebocytes de limule afin de déterminer la quantité d'endotoxine ou de 1,3-β-D-glucane contenue dans le biofilm, les étapes suivantes étant exécutées auparavant :
a) dissolution complète du biofilm adhérant à la surface du système, au moyen d'un solvant qui libère intégralement l'endotoxine ou le 1,3-β-D-glucane contenus dans les micro-organismes et qui préserve pour l'essentiel l'activité chimique de ces substances ; et
b) prélèvement d'échantillons de liquide à partir de la totalité du solvant utilisé, le test au lysat d'amoebocytes de limule étant réalisé avec les échantillons de liquide afin de quantifier la charge en biofilm.

14. Méthode selon la revendication 13, **caractérisée en ce que** l'on répète la méthode jusqu'à ce que la charge en biofilm soit entièrement dissoute, c'est-à-dire jusqu'à ce que plus aucune trace d'endotoxine et/ou de 1,3-β-D-glucane ne soit détectable.

15. Méthode selon l'une des revendications 1 à 14, **caractérisée en ce qu'**on utilise de l'hypochlorite de sodium comme solvant.

16. Méthode selon l'une des revendications 1 à 15, **caractérisée en ce que** pour la dissolution du biofilm, on utilise un excès de solvant, de sorte que le processus de dissolution à température ambiante se termine dans un laps de temps inférieur à 60 minutes.

17. Méthode selon l'une des revendications 1 à 16, **caractérisée en ce qu'**on utilise du thiosulfate de sodium comme agent neutralisant.
